# EUROPÄISCHE PATENTSCHRIFT

(11) **EP 1 205 205 B1**
(45) Veröffentlichungstag und Bekanntmachung des Hinweises auf die Patenterteilung: **15.06.2005**
(21) Anmeldenummer: 01126897.6
(22) Anmeldetag: 12.11.2001
(51) Int. Cl.: A61M 16/06

(54) **Atemmaske mit Stirnstütze**
Respiratory mask with forehead support
Masque respiratoire muni d'un support frontal

(30) Priorität: 14.11.2000 DE 10056331
(43) Veröffentlichungstag der Anmeldung: 15.05.2002
(60) Teilanmeldung: 04022930.4
(73) Patentinhaber: Weinmann Geräte für Medizin GmbH & Co. KG, 22525 Hamburg (DE)
(72) Erfinder: Eifler, Martin, 25358 Horst (DE); Schulz, Gerd, 22869 Schenefeld (DE)
(74) Vertreter: Klickow, Hans-Henning, Dr.-Ing.

## Beschreibung

Die Erfindung betrifft eine Atemmaske mit Vorrichtung zur Abstützung der Atemmaske im Bereich einer Stirn eines Patienten, die mindestens ein im Bereich mindestens eines Halterungsschenkels der Atemmaske angeordnetes und über eine Verstelleinrichtung mit dem Halterungsschenkel verbundenes Stützelement aufweist und bei der die Verstelleinrichtung eine Längsführung in Richtung einer Längsachse des Halterungsschenkels und eine Querführung für die Positionierung des Stützelementes im wesentlichen quer zur Längsachse aufweist.

Derartige Stützelemente mit Verstelleinrichtung dienen dazu, eine an eine jeweilige Gesichtsgeometrie des Patienten angepaßte Abstützung einer Atemmaske im Bereich einer Stirn des Patienten vorzunehmen. Die bekannten Stirnstützen weisen entweder eine Verstellmöglichkeit auf, um eine Verstellung im wesentlichen senkrecht zur Stirn vorzunehmen und hierdurch den wirksamen Stirnabstand zu verändern, oder um eine Verstellung im wesentlichen parallel zur Gesichtsfläche vorzunehmen und hierdurch eine Anpassung an die jeweilige Stirnhöhe durchzuführen. Bekannt sind hierzu mechanische Verstellungen oder der gezielte Einbau von Elementen mit einer relativ zueinander unterschiedlichen geeigneten Dimensionierung.

Eine Stirnstütze mit einer verstelleinrichtung zur Vorgabe eines wirksamen Stirnabstandes ist beispielsweise in der US-PS 61 19 693 beschrieben. Es wird hier eine brückenartige Stirnstütze offenbart, die über zwei Tragarme mit der Atemmaske verbunden ist. Ein Querträger des Stützelementes, der zwei Stirnpolster trägt, weist im Bereich seiner den Stützarmen zugewandten Ausdehnung Rastungen auf. Durch eine Arretierung der Stützarme in unterschiedlichen Rastungen wird ein unterschiedlicher Abstand des Querträgers zur Atemmaske und damit ein unterschiedlich wirksamer Stirnabstand vorgegeben.

Aus der DE-U 299 23 126 ist es bereits bekannt, eine Stirnstütze über eine buchsenförmige Halterung mit einer tubusförmigen Tragsäule zu verbinden. Die Tragsäule ist hohl und verbindet die Atemmaske mit einem Beatmungsschlauch, der sich über den Kopf des Patienten hinwegerstreckt. Es ist somit keine separate Halterung für die Stirnstütze vorhanden, wie dies bei Atemmasken üblich ist, bei denen ein Anschluß des Beatmungsschlauches an die Atemmaske über einen im wesentlichen nach unten ausgerichteten Anschlußstutzen erfolgt. Die buchsenförmige Halterung der Stirnstütze ist zur Bereitstellung einer Höheneinstellung in einer Längsachse der Führungssäule verschieblich. Ein Stirnabstand wird durch zwei verschwenkbare Tragarme vorgegeben, die im Bereich ihrer Enden an einem Rückenschild der Stirnstütze arretierbar sind. Eine verschwenkung der Tragarme verursacht zumindest auch eine Abstandsveränderung der Stirnstütze zur Tragsäule.

Aus der US-A-5,042,478 ist eine weitere Atemmaske mit Stirnstütze bekannt. Auch hier ist eine Halterung der Stirnstütze außenseitig an einer tubusförmigen Tragsäule angeordnet. Aufgrund der Anatomie der menschlichen Nase ist die Tragsäule verschwenkbar mit einem Grundelement verbunden und in einem Benutzungszustand schräg zur lotrechten Richtung angeordnet. Eine Veränderung des Neigungswinkels der Tragsäule verändert sowohl die Höhenpositionierung als auch die Abstandspositionierung der Stirnstütze. Bei einer Verschiebung der Stirnstützenhalterung entlang der Tragsäule wird ebenfalls sowohl die Höhenpositionierung als auch die Abstandspositionierung zur Stirn des Patienten verändert.

Die bislang bekannten Vorrichtungen können im Hinblick auf ihren Einstellkomfort und ihre Bedienbarkeit noch nicht alle Anforderungen erfüllen, die aus einer Verwendung durch den Patienten selbst resultieren.

Aufgabe der vorliegenden Erfindung ist es daher, eine Vorrichtung der einleitend genannten Art derart zu konstruieren, daß eine vielseitige Einstellbarkeit bei gleichzeitig einfacher Bedienung bereit gestellt wird.

Diese Aufgabe wird erfindungsgemäß dadurch gelöst, daß für die verstelleinrichtung eine Arretierung in Richtung der Längsachse vorgesehen ist, daß für das Stützelement eine Arretierung quer zur Längsachse vorgesehen ist und daß die Querführung derart ausgebildet ist, daß die Verstelleinrichtung eine Führungsausnehmung aufweist, in die ein Schaft des Stützelementes eingeführt ist.

Die Konstruktion der Vorrichtung derart, daß sowohl eine Längsführung für die Verstelleinrichtung als auch eine separate Querführung für das Stützelement vorhanden sind, ermöglicht es, den Stirnabstand und die Stirnhöhe separat einzustellen. Insbesondere wird bei einer Verstellung in Richtung einer dieser Dimensionen nicht die Einstellung in . Richtung der anderen Dimension verändert. Ein Patient kann somit schrittweise eine Anpassung der Anordnung der Atemmaske an die Geometrie seines Gesichtes vornehmen, ohne über ein umfangreiches räumliches Vorstellungsvermögen verfügen zu müssen.

Zur Fixierung einer Adaption an eine jeweilige Stirnhöhe wird vorgeschlagen, daß für die Verstelleinrichtung eine Arretierung in Richtung der Längsachse vorgesehen ist.

Zur Fixierung einer Adaption an einen jeweiligen Stirnabstand wird vorgeschlagen, daß für das Stützelement eine Arretierung quer zur Längsachse vorgesehen ist.

Zur Erleichterung einer Einstellung ist vorgesehen, daß für die Führung der Verstelleinrichtung in Richtung der Längsachse eine Rastung vorgesehen ist.

Darüber hinaus ist auch daran gedacht, daß die Verstelleinrichtung in Richtung der Längsachse stufenlos verstellbar ist.

Eine weitere Bedienungserleichterung kann dadurch erreicht werden, daß für die Verstellung des Stützelementes quer zur Längsachse eine Rastung vorgesehen ist.

Auch im Hinblick auf diese Verstellmöglichkeit ist daran gedacht, daß das Stützelement quer zur Längsachse stufenlos verstellbar ist.

Eine weiche Abpolsterung der Stirn kann dadurch erfolgen, daß das Stützelement ein Stirnpolster trägt, das als ein Gelkissen ausgebildet ist.

Darüber hinaus ist daran gedacht, daß das Stützelement ein Stirnpolster trägt, das als ein elastomeres Polster ausgebildet ist.

Ebenfalls ist es möglich, daß das Stützelement ein Stirnpolster trägt, das als ein Schaumpolster ausgebildet ist.

Eine weitere Erhöhung der Einstellungsflexibilität kann dadurch erreicht werden, daß das Stützelement relativ zum Halterungsschenkel verschwenkbar angeordnet ist.

Ebenfalls ist daran gedacht, daß der Halterungsschenkel relativ zu einem Maskengrundkörper der Atemmaske verschwenkbar angeordnet ist.

Eine weitere Anpassung an einen individuellen Patienten kann dadurch erreicht werden, daß das Stirnpolster in frontaler Richtung verkippbar angeordnet ist.

Ein weiter gesteigerter Benutzungskomfort kann dadurch erreicht werden, daß das Stirnpolster seitlich kippbar angeordnet ist.

Eine besonders gute Anpassung an eine Stirnkontur wird dadurch unterstützt, daß das Stirnpolster als ein elastomeres Band ausgebildet ist, das von einer gabelartigen Halterung positioniert ist.

Gemäß einer weiteren Ausführungsvariante ist daran gedacht, daß das Stirnpolster als ein elastomeres Band ausgebildet ist, das einteilig mit einer gabelartigen Halterung konstruiert ist.

In den Zeichnungen sind Ausführungsbeispiele der Erfindung schematisch dargestellt. Es zeigen:
- Fig. 1: Eine perspektivische Darstellung einer Atemmaske mit einem Stützelement, das sowohl in Richtung einer Längsachse eines Halterungsschenkels als auch quer zur Längsachse positionierbar ist,
- Fig. 2: eine Ansicht gemäß Blickrichtung II in Fig. 1,
- Fig. 3: eine Ansicht gemäß Blickrichtung III in Fig. 1,
- Fig. 4: eine perspektivische Darstellung gemäß Blickrichtung IV in Fig. 3,
- Fig. 5: eine Explosionsdarstellung des Stützelementes mit Stirnpolster und Verstelleinrichtung,
- Fig. 6: eine Darstellung der Anordnung gemäß Blickrichtung VI in Fig. 5,
- Fig. 7: eine vergrößerte perspektivische Darstellung des Stützelementes,
- Fig. 8: eine Seitenansicht des Stützelementes gemäß Blickrichtung VIII in Fig. 7,
- Fig. 9: eine vergrößerte perspektivische Darstellung des Stirnpolsters,
- Fig. 10: eine vergrößerte perspektivische Darstellung der Verstelleinrichtung,
- Fig. 11: eine Darstellung der Verstelleinrichtung gemäß Blickrichtung XI in Fig. 10,
- Fig. 12: die Verstellvorrichtung gemäß Blickrichtung XII in Fig. 10,
- Fig. 13: eine Seitenansicht gemäß Blickrichtung XIII in Fig. 1,
- Fig. 14: eine weitere perspektivische Darstellung der Verstelleinrichtung,
- Fig. 15: einen Längsschnitt gemäß Schnittlinie XV-XV in Fig. 11 nach einer Drehung um 90°,
- Fig. 16: eine perspektivische Darstellung der verstelleinrichtung gemäß Blickrichtung XVI in Fig. 14,
- Fig. 17: eine weitere perspektivische Darstellung der Verstelleinrichtung,
- Fig. 18: eine weitere perspektivische Darstellung des Stützelementes,
- Fig. 19: eine weitere perspektivische Darstellung des Stirnpolsters,
- Fig. 20: eine perspektivische Darstellung gemäß Blickrichtung XX in Fig. 19 nach einer Drehung um 90°,
- Fig. 21: eine Ansicht gemäß Blickrichtung XXI in Fig. 19,
- Fig. 22: einen Querschnitt gemäß Schnittlinie XXII-XXII in Fig. 19,
- Fig. 23: eine Prinzipdarstellung einer brückenartigen Stirnstütze und
- Fig. 24: eine gegenüber Fig. 23 abgewandelte Konstruktion einer brückenförmigen Stirnstütze in einteiliger Ausführung.

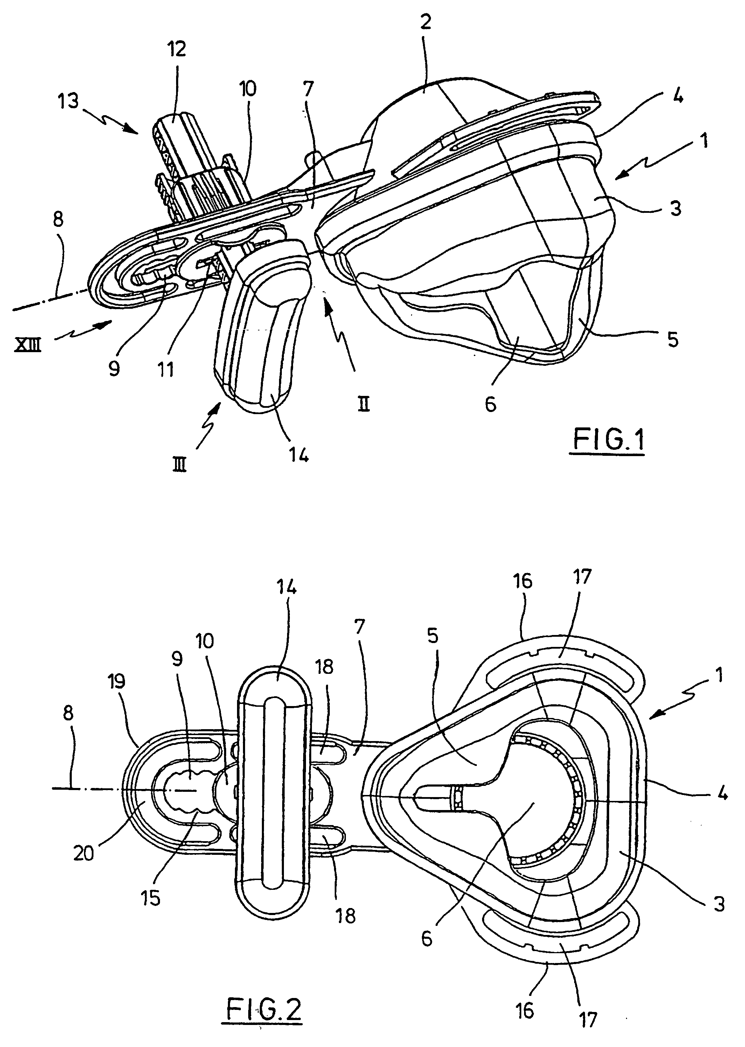

Fig. 1 zeigt eine perspektivische Darstellung einer Atemmaske (1), die im wesentlichen aus einem Maskengrundkörper (2) aus einem festen Material und einem Maskenpolster (3) aus einem weichen Material besteht. Das Maskenpolster (3) kann über einen Polstersockel (4) auf einen Rand des Maskengrundkörpers (2) aufgesteckt sein. Das Maskenpolster (3) begrenzt mit Dichtungslippen (5) eine Atemöffnung (6), in die bei einer Benutzung durch einen Patienten eine Nase des Patienten eingeführt wird.

Am Maskengrundkörper (2) ist ein Halterungsschenkel (7) befestigt, der sich mit einer Längsachse (8) im wesentlichen parallel zu einer von der Atemöffnung (6) aufgespannten Ebene erstreckt. Der Halterungsschenkel (7) weist eine Montageöffnung (9) auf, in die eine Verstelleinrichtung (10) eingesetzt ist. Die Verstelleinrichtung (10) weist quer zur Längsachse (8) eine Führungsausnehmung (11) auf, in die ein Schaft (12) eines Stützelementes (13) eingeführt ist. Das Stützelement (13) trägt ein Stirnpolster (14).

Aus der Darstellung in Fig. 2 ist erkennbar, daß sich das Stirnpolster (14) im wesentlichen quer zur Längsachse (8) erstreckt. Ebenfalls ist zu erkennen, daß die Montageöffnung (9) ähnlich zu einem Längsschlitz ausgebildet ist, der sich in Richtung der Längsachse (8) erstreckt. Gemäß der Ausführungsform in Fig. 1 und Fig. 2 weist die Montageöffnung (9) mehrere Rastungen (15) auf, um eine stufenweise definierte Positionierung der Verstelleinrichtung (10) in Richtung der Längsachse (8) zu unterstützen. Fig. 2 veranschaulicht ebenfalls, daß am Maskengrundkörper (2) seitlich zwei Flansche (16) angeformt sind, die jeweils mit schlitzförmigen Ausnehmungen (17) versehen sind.

Alternativ zu einer Verwendung von Rastungen (15) ist es auch denkbar, für die Positionierung der Verstelleinrichtung (10) in Richtung der Längsachse (8) mehrere Aufnahmen im Bereich des Halterungsschenkels (7) anzuordnen und die Verstelleinrichtung (10) jeweils in eine dieser Aufnahmen einzusetzen.

Im Bereich des Halterungsschenkels (7) sind seitlich zwei Ausnehmungen (18) und im Bereich eines Endsegmentes (19) ist eine hufeisenartige Ausnehmung (20) angeordnet.

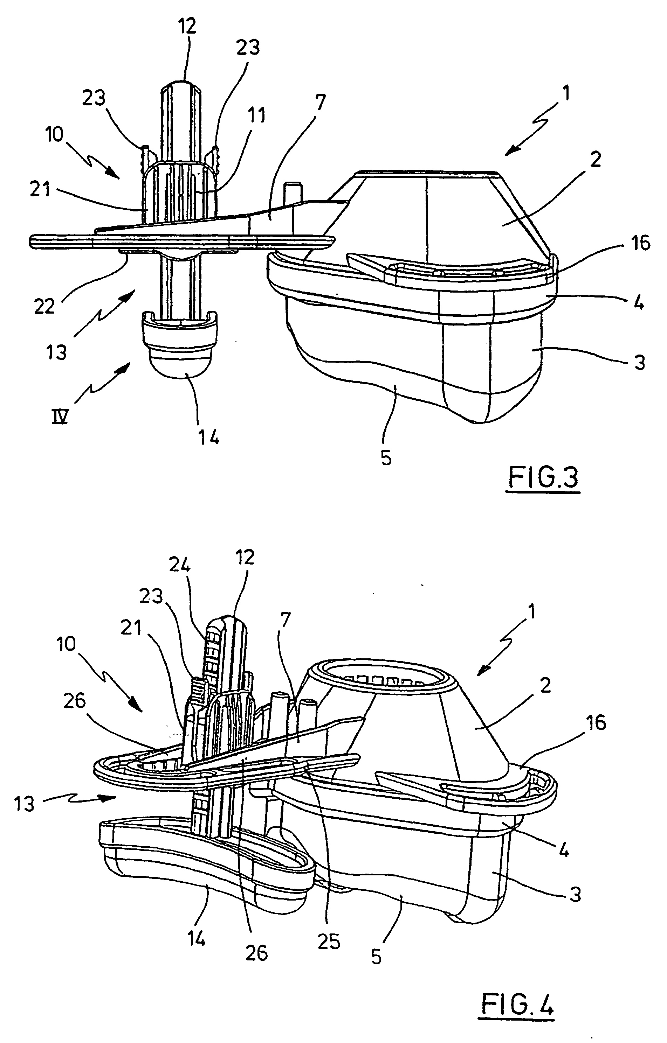

Aus Fig. 3 ist erkennbar, daß die Verstelleinrichtung (10) mit einem Grundkörper (21) durch die Montageöffnung (9) hindurchragt und mit einer Platte (22) auf dem Halterungsschenkel (7) aufliegt. Der Grundkörper (21) weist federnd gelagerte Klemmstege (23) auf, die den Schaft (12) des Stützelementes (13) innerhalb der Führungsausnehmung (11) der Verstelleinrichtung (10) fixieren.

Die perspektivische Darstellung in Fig. 4 veranschaulicht, daß der Schaft (12) im Bereich seiner den Klemmstegen (23) zugewandten Seiten mit einer Rastung (24) versehen ist, um auch hier eine definierte und schrittweise Positionierung des Stützelementes (13) zu ermöglichen.

Fig. 4 veranschaulicht ebenfalls, daß der Halterungsschenkel (7) im wesentlichen aus einer Schenkelplatte (25) sowie Stützstegen (26) besteht, die im wesentlichen senkrecht zur Schenkelplatte (25) angeordnet sind und zu einer erhöhten Biegesteifigkeit beitragen. Die Stützstege (26) verjüngen sich in eine dem Maskengrundkörper (2) abgewandte Richtung und sind im Bereich ihrer verdickten Enden am Maskengrundkörper (2) angeformt.

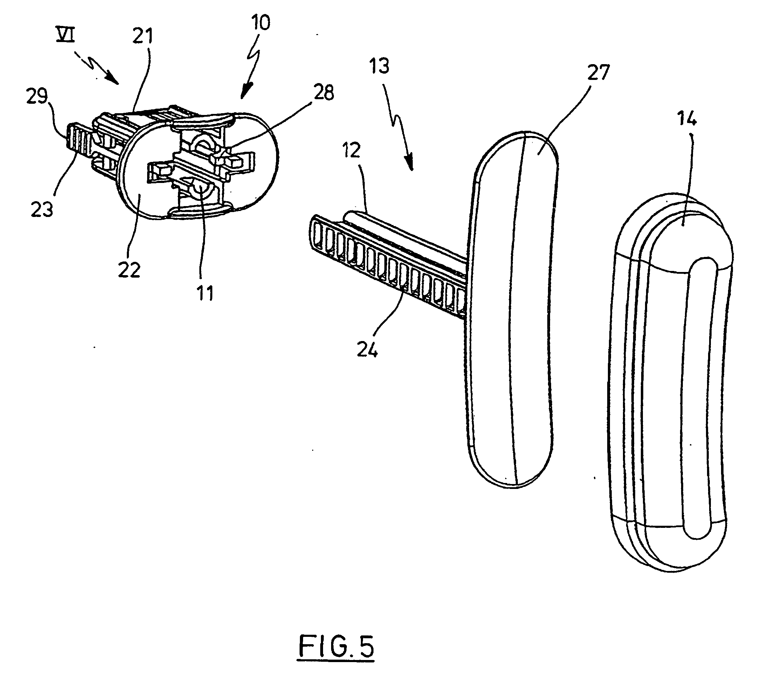

Fig. 5 veranschaulicht in einer Explosionsdarstellung die Konstruktion der Verstelleinrichtung (10) und des Stützelementes (13). Insbesondere ist erkennbar, daß das Stützelement (13) eine Stützplatte (27) aufweist, auf die das Stirnpolster (14) aufgesteckt wird. Ebenfalls ist aus Fig. 5 zu erkennen, daß die Klemmstege (23) mit Rastvorsprüngen (28) versehen sind, die in die Rastungen (24) eingreifen können. Bei einem manuellen Druck auf Bedienflächen (29) der Klemmstege (23) werden die Rastvorsprünge (28) aus der Rastung (24) herausgeführt.

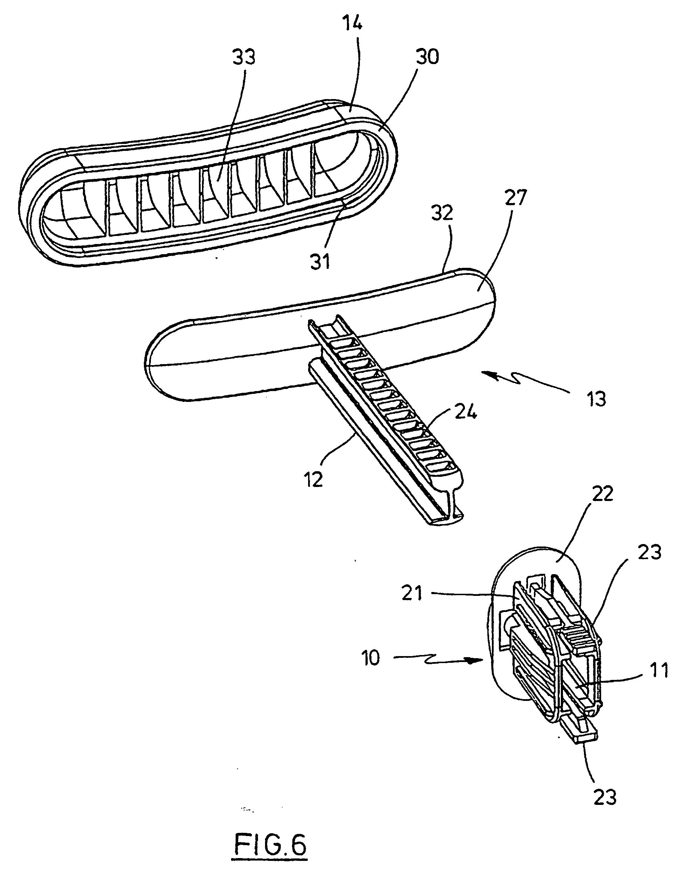

Fig. 6 veranschaulicht, daß das Stirnpolster (14) im Bereich eines Randes (30) mit einem U-Profil (31) versehen ist, das auf einen Rand (32) der Stützplatte (27) aufsteckbar ist. Darüber hinaus weist das Stirnpolster (14) zur Bereitstellung einer ausreichenden Eigenstabilität innere Querstege (33) auf, die bei einer Ausbildung des Stirnpolsters (14) aus einem elastomeren Material zu einem guten Kompromiß zwischen einer ausreichenden Formstabilität und einer ausreichenden Nachgiebigkeit beitragen.

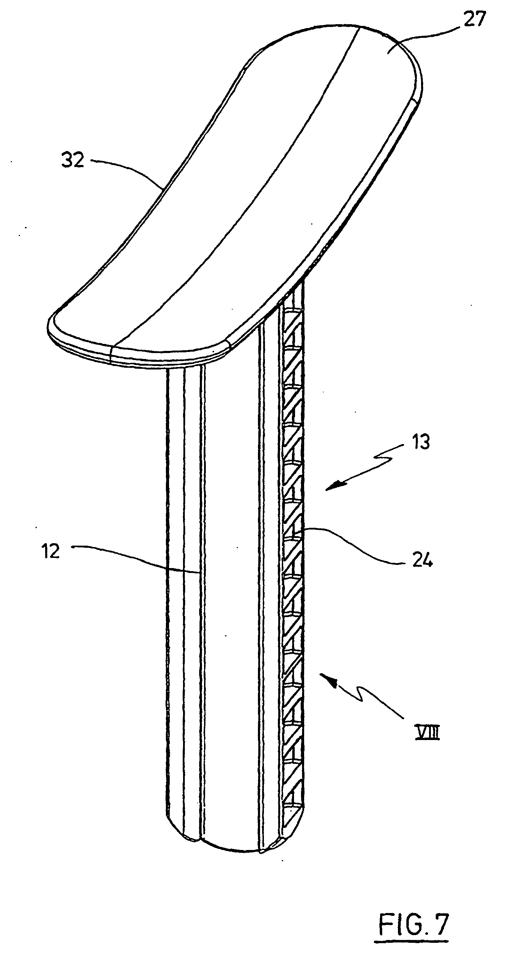

Die Darstellung des Stützelementes (13) in Fig. 7 veranschaulicht, daß durch die Rastung (24) eine Vielzahl von Rasterfächern ausgebildet werden, die jeweils zur Aufnahme der Rastvorsprünge (28) der Klemmstege (23) vorgesehen sind. Ebenfalls ist erkennbar, daß die Stützplatte (27) leicht gewölbt ist, um eine Anpassung an die Kontur einer menschlichen Stirn zu gewährleisten.

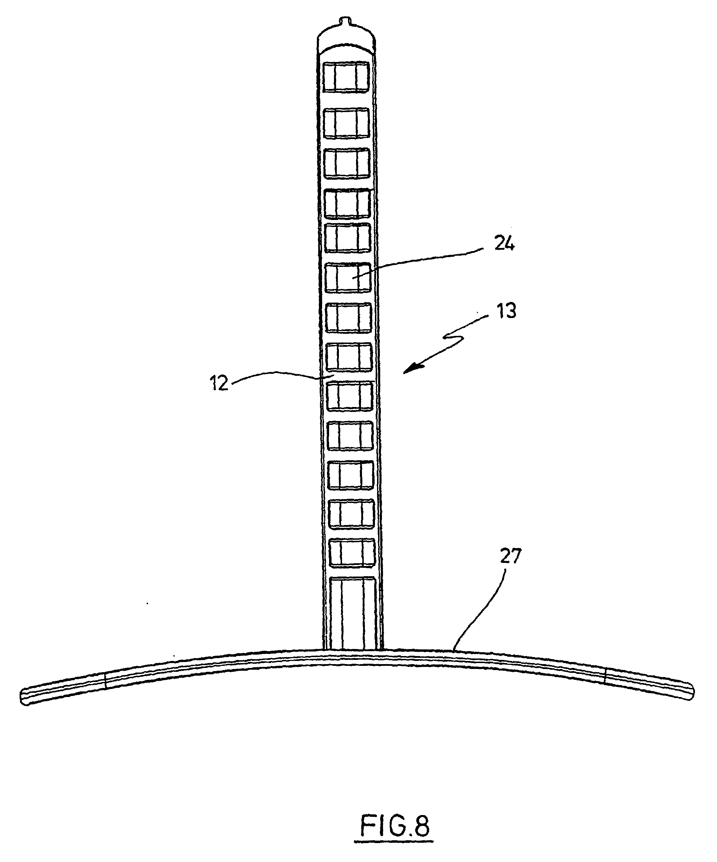

Die Wölbung der Stützplatte (27) sowie die Anordnung der Rastung (24) entlang der Längserstreckung des Schaftes (12) wird in Fig. 8 noch einmal veranschaulicht.

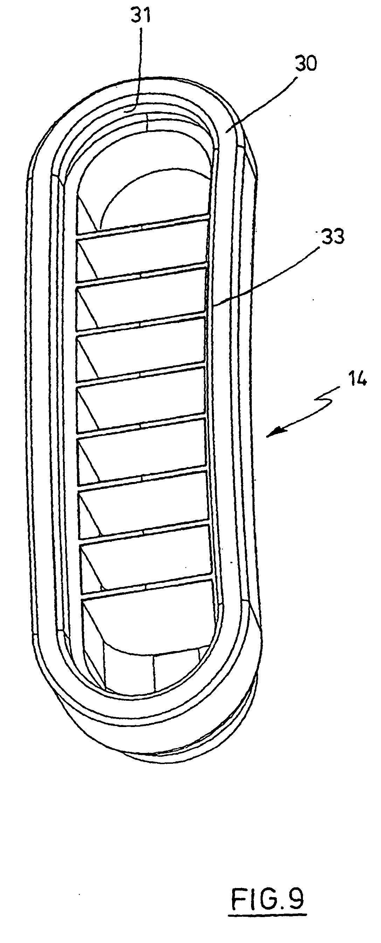

Aus der Darstellung in Fig. 9 ist der Aufbau des Stirnpolsters (14) in größerer Detailliertheit zu erkennen. Das Stirnpolster (14) weist eine haubenartige Querschnittgestaltung auf und von den Querstegen (33) werden sowohl Längsseiten des Stirnpolsters (14) relativ zueinander abgestützt als auch eine Versteifung im Bereich einer dem Rand (30) abgewandten Abschlußrundung hervorgerufen.

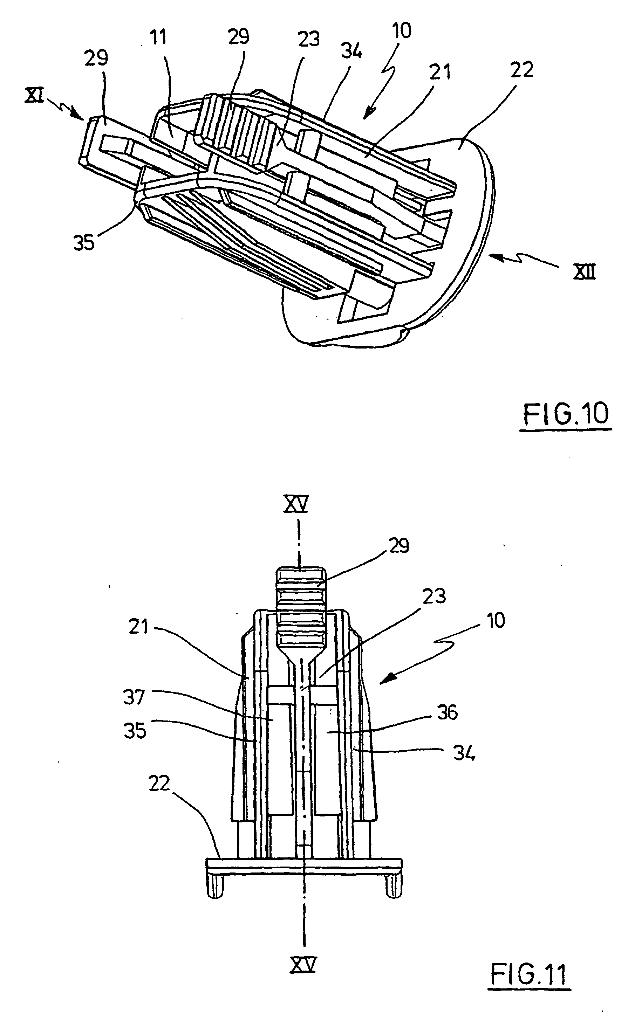

Die vergrößerte perspektivische Darstellung in Fig. 10 zeigt die Verstelleinrichtung (10) in stärkerer Detailliertheit. Es ist zu erkennen, daß der Grundkörper (21) im wesentlichen aus zwei Platten (34, 35) besteht, zwischen denen die Klemmstege (23) angeordnet sind.

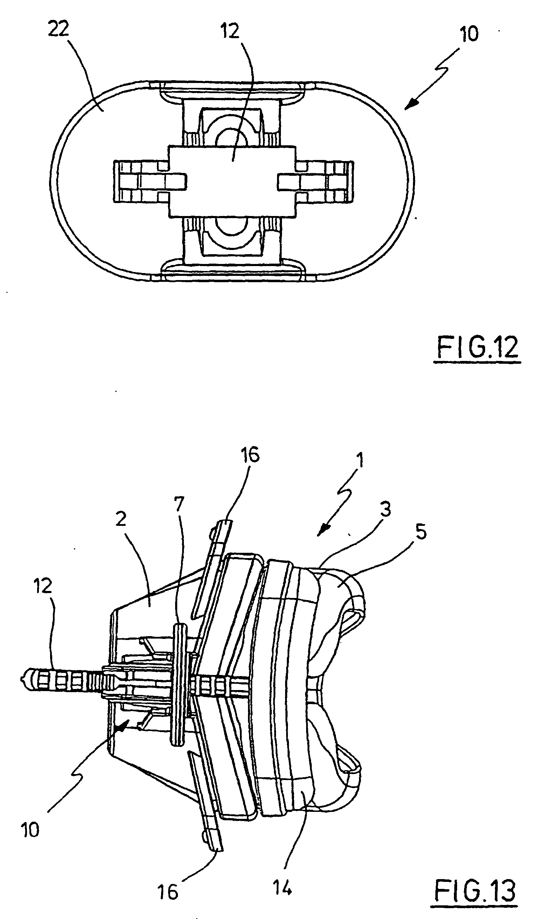

Aus Fig. 11 ist zu erkennen, daß an den Platten (34, 35) Querstege (36, 37) angeordnet sind, die sich in Richtung auf die Klemmstege (23) erstrecken.

Fig. 12 veranschaulicht die Kombination der Verstelleinrichtung (10) mit dem Schaft (12). Fig. 13 zeigt in einer weiteren Seitenansicht als Zusammenbaudarstellung die Kombination der Atemmaske (1) mit einem Stirnpolster (14), das über einen Schaft (12) von einer Verstelleinrichtung (10) im Bereich des Halterungsschenkels (7) fixiert ist.

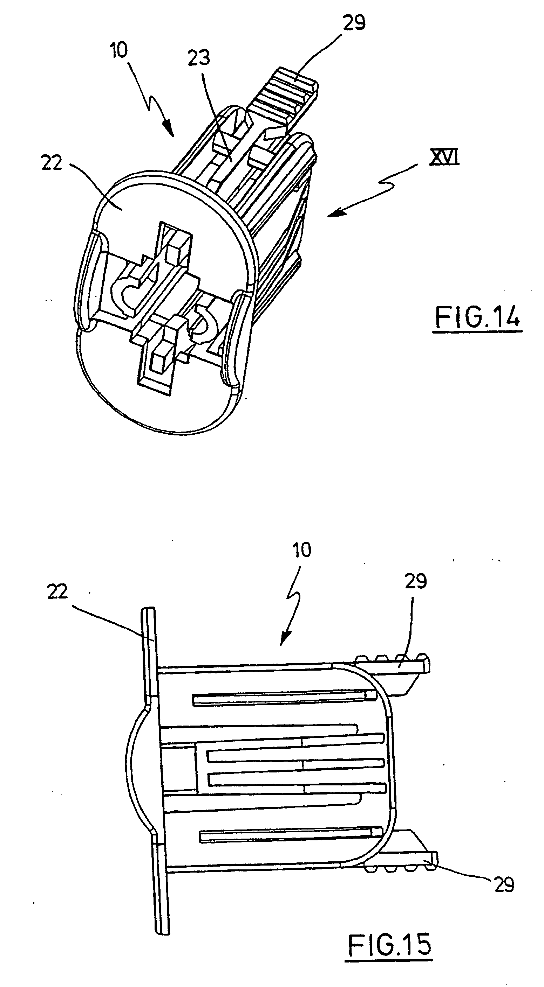

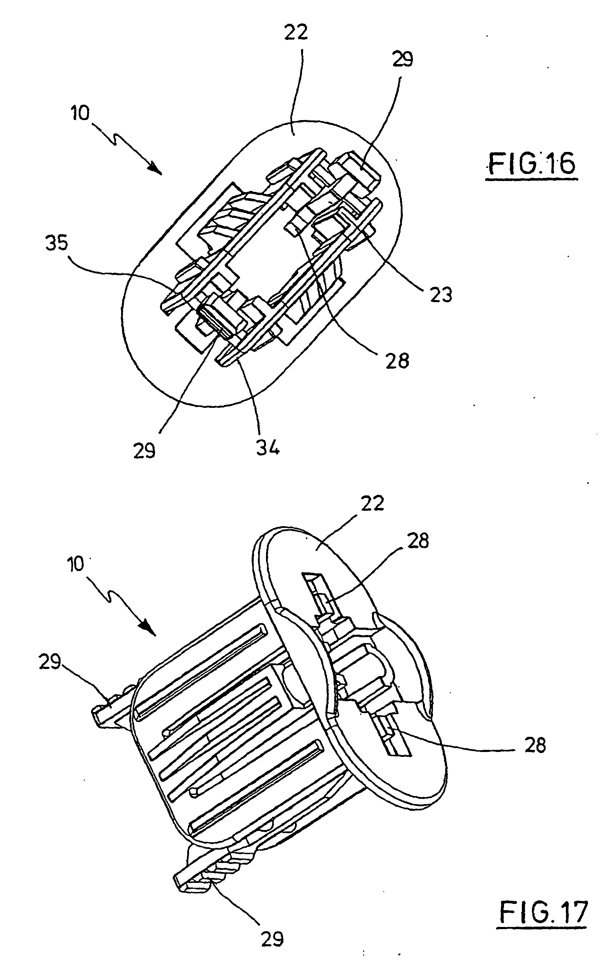

Fig. 14 und Fig. 15 zeigen nochmals den Aufbau der Verstelleinrichtung (10). Weitere Darstellungen der Verstelleinrichtung (10) finden sich auch in Fig. 16 und in Fig. 17.

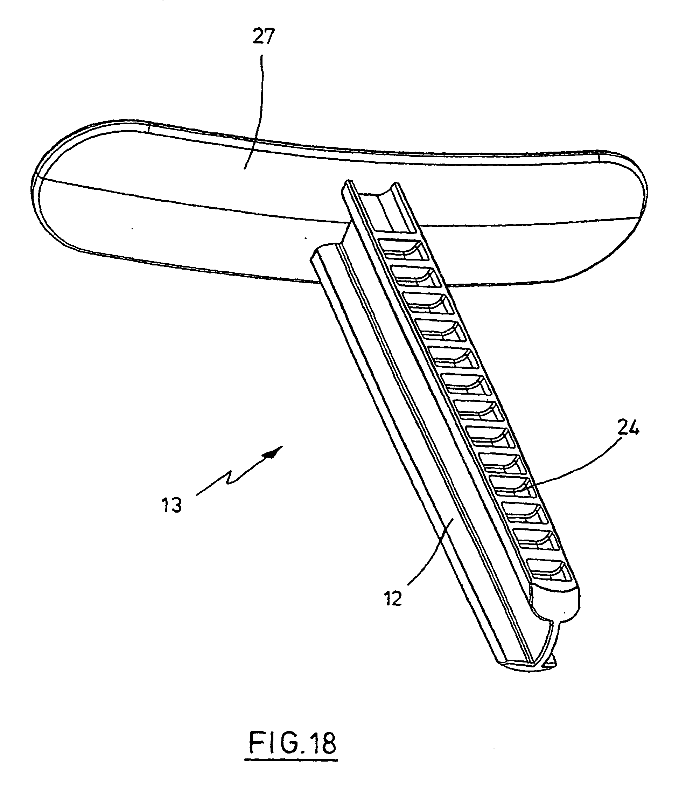

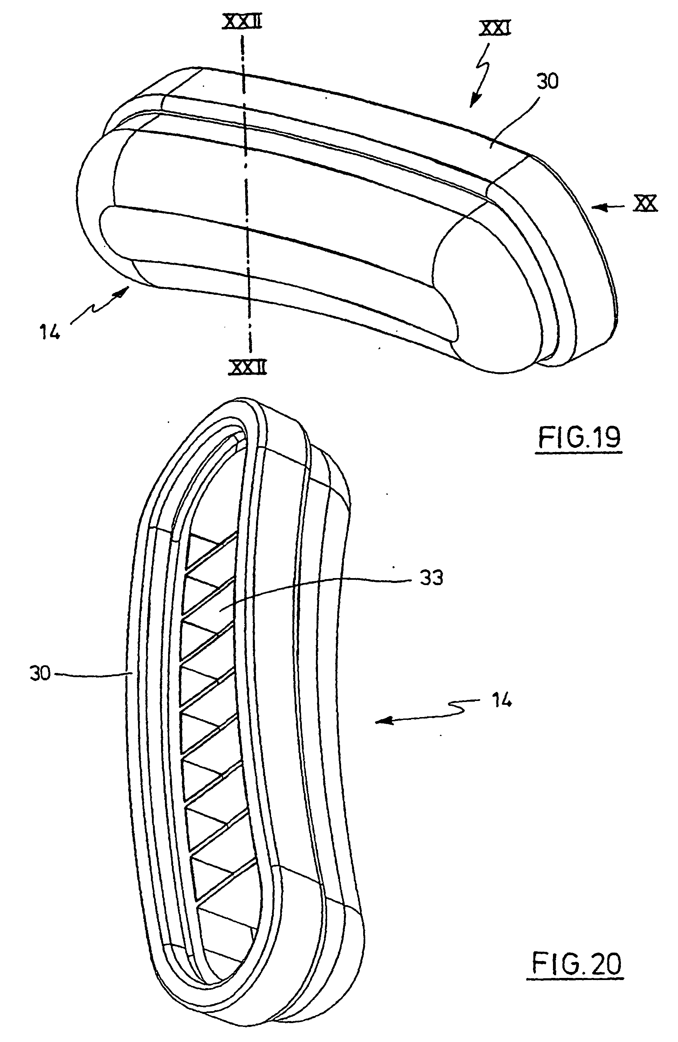

Fig. 18 zeigt in einer weiteren perspektivischen Darstellung nochmals das Stützelement (13), wobei insbesondere erkennbar ist, daß der Schaft (12) ein Querschnittprofil ähnlich zu einem I aufweist. Fig. 19 und Fig. 20 zeigen nochmals in weiteren perspektivischen Darstellungen die Gestaltung des Stirnpolsters (14).

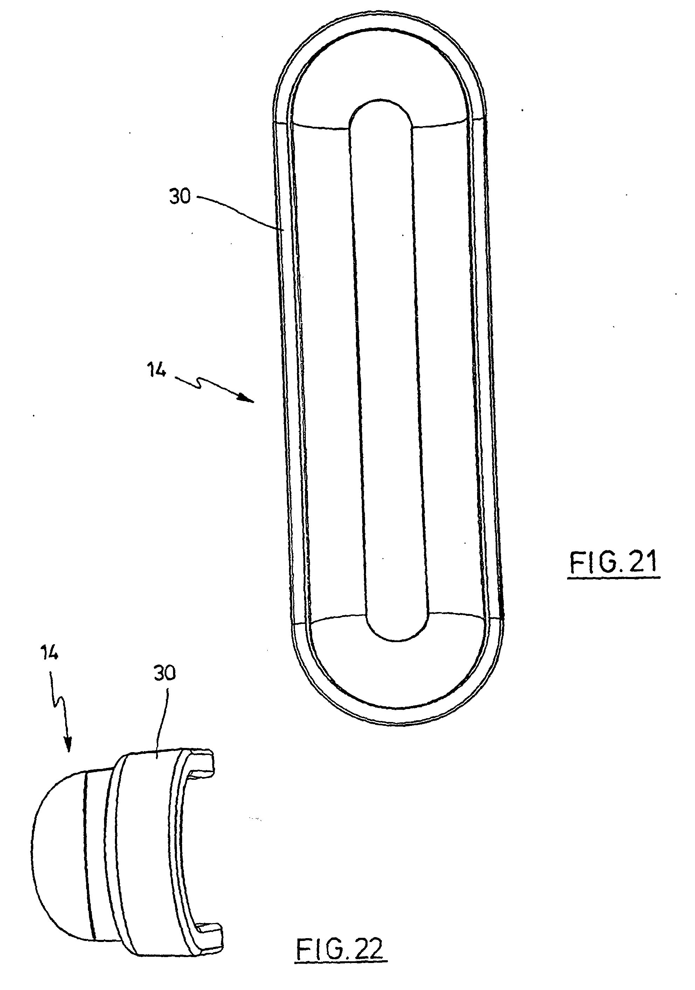

Fig. 21 veranschaulicht, daß das Stirnpolster (14) im wesentlichen eine symmetrische Gestaltung zu seiner Längsachse aufweist. Fig. 22 veranschaulicht die gerundete äußere Konturierung des Stirnpolsters (14).

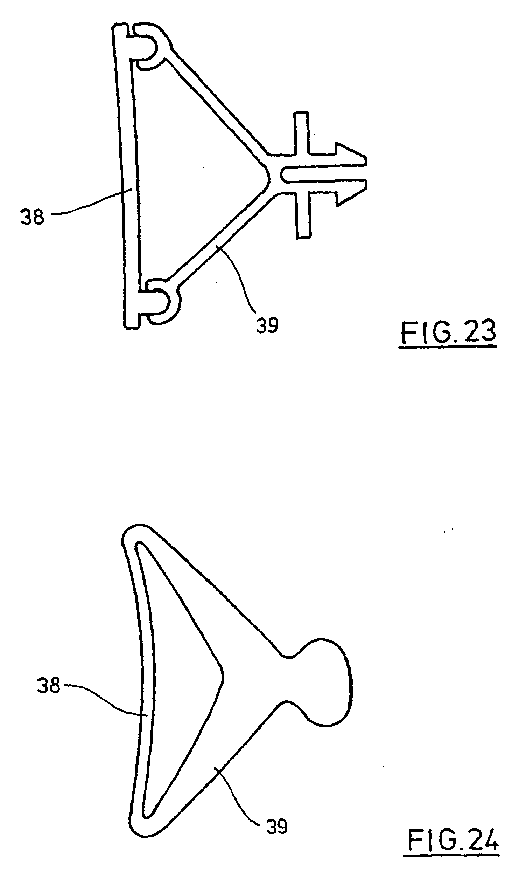

Fig. 23 zeigt eine modifizierte Ausführungsform, bei der ein elastomeres Band (38) von einer gabelartigen Halterung (39) positioniert ist. Das elastomere Band (38) kann sich der Stirnkontur und der Stirnneigung anpassen.

Fig. 24 zeigt eine zur Fig. 23 modifizierte Ausführungsform, bei der das elastomere Band (38) und die Halterung (39) einteilig ausgeführt sind. Als Werkstoff kann z.B. Schaum oder ein Hohlkörper verwendet werden. Ein typischer Einstellvorgang erfolgt derart, daß der Patient zunächst die Atemmaske (1) im Bereich seiner Nase positioniert und in einem ersten Schritt eine Anpassung an die Stirnhöhe durch eine Verschiebung der Verstelleinrichtung (10) innerhalb der Montageöffnung (9) in Richtung der Längsachse (8) durchführt. Über die Rastung (15) wird eine definierte Positionierung unterstützt. Nach einer geeigneten Anordnung und Arretierung der Verstelleinrichtung (10) innerhalb der Montageöffnung (9) wird eine Anpassung an den Stirnabstand durch ein Herausziehen des Schaftes (12) aus der Führungsausnehmung (11) bzw. durch ein Hineinschieben des Schaftes (12) in die Führungsausnehmung (11) durchgeführt. Vor dieser Positionierung werden durch einen Druck auf die Bedienflächen (29) der Klemmstege (23) die Rastvorsprünge (28) aus den Vertiefungen der Rastung (24) herausgeschwenkt. Nach einer geeigneten Positionierung des Schaftes (12) des Stützelementes (13) werden die Bedienflächen (29) losgelassen und die Rastvorsprünge (28) federn in die Rastung (24) zurück. Hierdurch ist das Stützelement (13) in seiner aktuellen Positionierung arretiert.

Sollte eine nachträgliche Veränderung der gewählten Positionierungen erforderlich sein, so kann sowohl eine Änderung der Stirnhöheneinstellung als auch eine Änderung der Stirnabstandseinstellung ohne Rückwirkung auf die jeweils andere Einstellung erfolgen. Die erläuterten Arretierungen vermeiden eine ungewollte Veränderung der Positionierung des Stützelementes (13) während der Benutzung.

## Patentansprüche

1. Atemmaske (1) mit Vorrichtung zur Abstützung der Atemmaske (1) im Bereich einer Stirn eines Patienten, die mindestens ein im Bereich mindestens eines Halterungsschenkels (7) der Atemmaske (1) angeordnetes und über eine Verstelleinrichtung (10) mit dem Halterungsschenkel (7) verbundenes Stützelement (13) aufweist und bei der die Verstelleinrichtung (10) eine Längsführung in Richtung einer Längsachse (8) des Halterungsschenkels (7) und eine Querführung für die Positionierung des Stützelementes (13) im wesentlichen quer zur Längsachse (8) aufweist, **dadurch gekennzeichnet, daß** für die Verstelleinrichtung (10) eine Arretierung in Richtung der Längsachse (8) vorgesehen ist, daß für das Stützelement (13) eine Arretierung quer zur Längsachse (8) vorgesehen ist und daß die Querführung derart ausgebildet ist, daß die Verstelleinrichtung (10) eine Führungsausnehmung (11) aufweist, in die ein Schaft (12) des Stützelementes (13) eingeführt ist.

2. Atemmaske nach Anspruch 1, **dadurch gekennzeichnet, daß** für die Führung der Verstelleinrichtung (10) in Richtung der Längsachse (8) eine Rastung vorgesehen ist.

3. Atemmaske nach Anspruch 1 oder 2, **dadurch gekennzeichnet, daß** die Verstelleinrichtung (10) in Richtung der Längsachse (8) stufenlos verstellbar ist.

4. Atemmaske nach einem der Ansprüche 1 bis 3, **dadurch gekennzeichnet, daß** für die Verstellung des Stützelementes (13) quer zur Längsachse (8) eine Rastung vorgesehen ist.

5. Atemmaske nach einem der Ansprüche 1 bis 3, **dadurch gekennzeichnet, daß** das Stützelement (13) quer zur Längsachse (8) stufenlos verstellbar ist.

6. Atemmaske nach einem der Ansprüche 1 bis 5, **dadurch gekennzeichnet, daß** das Stützelement (13) ein Stirnpolster (14) trägt, das als ein Gelkissen ausgebildet ist.

7. Atemmaske nach einem der Ansprüche 1 bis 5, **dadurch gekennzeichnet, daß** das Stützelement (13) ein Stirnpolster (14) trägt, das als ein elastomeres Polster ausgebildet ist.

8. Atemmaske nach einem der Ansprüche 1 bis 5, **dadurch gekennzeichnet, daß** das Stützelement (13) ein Stirnpolster (14) trägt, das als ein Schaumpolster ausgebildet ist.

9. Atemmaske nach einem der Ansprüche 1 bis 8, **dadurch gekennzeichnet, daß** das Stützelement (13) relativ zum Halterungsschenkel (7) verschwenkbar angeordnet ist.

10. Atemmaske nach einem der Ansprüche 1 bis 9, **dadurch gekennzeichnet, daß** der Halterungsschenkel (7) relativ zu einem Maskengrundkörper (2) der Atemmaske (1) verschwenkbar angeordnet ist.

11. Atemmaske nach einem der Ansprüche 6 bis 10, **dadurch gekennzeichnet, daß** das Stirnpolster (14) in frontaler Richtung verkippbar angeordnet ist.

12. Atemmaske nach einem der Ansprüche 6 bis 11, **dadurch gekennzeichnet, daß** das Stirnpolster (14) seitlich kippbar angeordnet ist.

## Claims

1. Respiratory mask (1) having a device for supporting the respiratory mask (1) in the region of a patient's forehead, comprising at least one supporting element (13) arranged in the region of at least one retaining arm (7) of the respiratory mask (1) and connected by an adjusting device (10) to the retaining arm (7), and wherein the adjusting device (10) comprises a longitudinal guide in the direction of a longitudinal axis (8) of the retaining arm (7) and a transverse guide for positioning the supporting element (13) substantially transversely to the longitudinal axis (8), **characterised in that** a locking device is provided for the adjusting device (10) in the direction of the longitudinal axis (8), **in that** a locking device is provided for the supporting element (13) transversely to the longitudinal axis (8), and **in that** the transverse guide is constructed in such a way that the adjusting device (10) comprises a guide recess (11) into which a shaft (12) of the supporting element (13) is introduced.

2. Respiratory mask according to claim 1, **characterised in that** a latching device is provided for guiding the adjusting device (10) in the direction of the longitudinal axis (8).

3. Respiratory mask according to claim 1 or 2, **characterised in that** the adjusting device (10) is continuously adjustable in the direction of the longitudinal axis (8).

4. Respiratory mask according to any one of claims 1 to 3, **characterised in that** a latching device is provided for adjusting the supporting element (13) transversely to the longitudinal axis (8).

5. Respiratory mask according to any one of claims 1 to 3, **characterised in that** the supporting element (13) is continuously adjustable transversely to the longitudinal axis (8).

6. Respiratory mask according to any one of claims 1 to 5, **characterised in that** the supporting element (13) carries a forehead cushion (14) which is constructed as a gel cushion.

7. Respiratory mask according to any one of claims 1 to 5, **characterised in that** the supporting element (13) carries a forehead cushion (14) which is constructed as an elastomeric cushion.

8. Respiratory mask according to any one of claims 1 to 5, **characterised in that** the supporting element (13) carries a forehead cushion (14) which is constructed as a foam cushion.

9. Respiratory mask according to any one of claims 1 to 8, **characterised in that** the supporting element (13) is pivotally arranged relative to the retaining arm (7).

10. Respiratory mask according to any one of claims 1 to 9, **characterised in that** the retaining arm (7) is pivotally arranged relative to a basic body (2) of the respiratory mask (1).

11. Respiratory mask according to any one of claims 6 to 10, **characterised in that** the forehead cushion (14) is tiltably arranged in the frontal direction.

12. Respiratory mask according to any one of claims 6 to 11, **characterised in that** the forehead cushion (14) is laterally tiltably arranged.

## Revendications

1. Masque respiratoire (1) avec un dispositif d'appui du masque respiratoire (1) dans la région du front d'un patient, qui présente au moins un organe d'appui (13) agencé dans la région d'au moins un bras de fixation (7) du masque respiratoire (1), et relié par un dispositif d'ajustement (10) au bras de fixation (7), et dans lequel le dispositif d'ajustement (10) présente un guide longitudinal dans la direction d'un axe longitudinal (8) du bras de fixation (7) et un guide transversal pour le positionnement de l'organe d'appui (13) essentiellement perpendiculairement à l'axe longitudinal (8), **caractérisé en ce qu'**un arrêt est prévu pour le dispositif d'ajustement (10) dans la direction de l'axe longitudinal (8), **en ce qu'**une butée est prévue pour l'organe d'appui (13) perpendiculairement à l'axe longitudinal (8), et **en ce que** le guide transversal est réalisé d'une manière telle que le dispositif d'ajustement (10) présente un logement de guidage (11) dans lequel est introduite une tige (12) de l'organe d'appui (13).

2. Masque respiratoire selon la revendication 1, **caractérisé en ce qu'**un organe d'enclenchement est prévu pour guider le dispositif d'ajustement (10) en direction de l'axe longitudinal (8).

3. Masque respiratoire selon la revendication 1 ou 2, **caractérisé en ce que** le dispositif d'ajustement (10) est ajustable en continu en direction de l'axe longitudinal (8).

4. Masque respiratoire selon l'une des revendications 1 à 3, **caractérisé en ce qu'**un organe d'enclenchement est prévu pour ajuster le dispositif d'appui (13) perpendiculairement à l'axe longitudinal (8).

5. Masque respiratoire selon l'une des revendications 1 à 3, **caractérisé en ce que** l'organe d'appui (13) est ajustable en continu perpendiculairement à l'axe longitudinal (8).

6. Masque respiratoire selon l'une des revendications 1 à 5, **caractérisé en ce que** l'organe d'appui (13) porte un rembourrage frontal (14) qui est réalisé sous la forme d'un coussin de gel.

7. Masque respiratoire selon l'une des revendications 1 à 5, **caractérisé en ce que** l'organe d'appui (13) porte un rembourrage frontal (14) qui est réalisé sous la forme d'un rembourrage en élastomère.

8. Masque respiratoire selon l'une des revendications 1 à 5, **caractérisé en ce que** l'organe d'appui (13) porte un rembourrage frontal (14) qui est réalisé sous la forme d'un rembourrage en mousse.

9. Masque respiratoire selon l'une des revendications 1 à 8, **caractérisé en ce que** l'organe d'appui (13) est agencé à pivotement par rapport au bras de fixation (7).

10. Masque respiratoire selon l'une des revendications 1 à 9, **caractérisé en ce que** le bras de fixation (7) est agencé à pivotement par rapport à un corps de base (2) du masque respiratoire (1).

11. Masque respiratoire selon l'une des revendications 6 à 10, **caractérisé en ce que** le rembourrage frontal (14) est agencé de manière basculante dans la direction frontale.

12. Masque respiratoire selon l'une des revendications 6 à 11, **caractérisé en ce que** le rembourrage frontal (14) est agencé de manière basculante dans la direction latérale.
